(19) Europäisches Patentamt
European Patent Office
Office européen des brevets

(11) Publication number: **0 414 151 B1**

(12) # EUROPEAN PATENT SPECIFICATION

(45) Date of publication of patent specification: **15.03.95**

(51) Int. Cl.$^6$: **C12N 15/16**, C07K 14/00, C12P 21/02, C12N 5/10

(21) Application number: **90115815.4**

(22) Date of filing: **17.08.90**

The file contains technical information submitted after the application was filed and not included in this specification

(54) **Production of human nerve growth factor proteins.**

(30) Priority: **21.08.89 JP 212980/89**
**20.12.89 JP 328198/89**
**13.04.90 JP 96252/90**
**07.06.90 JP 147392/90**

(43) Date of publication of application:
**27.02.91 Bulletin 91/09**

(45) Publication of the grant of the patent:
**15.03.95 Bulletin 95/11**

(84) Designated Contracting States:
**AT BE CH DE DK ES FR GB GR IT LI LU NL SE**

(56) References cited:
**EP-A- 0 121 338**

**NATURE. vol. 303, 30 June 1983, London, GB, pages 821 - 825; ULLRICH, A. et al.: "Human beta-nerve growth factor gene sequence highly homologous to that of mouse"**

(73) Proprietor: **Takeda Chemical Industries, Ltd.**
**1-1, Doshomachi 4-chome**
**Chuo-ku, OSAKA (JP)**

(72) Inventor: **Kakinuma, Atsushi**
**C2-505, 3 Takenodai**
**Nagaokakyo,**
**Kyoto 617 (JP)**
Inventor: **Nakahama, Kazuo**
**15-59, Nishinokyo**
**Nagaokakyo,**
**Kyoto 617 (JP)**
Inventor: **Yoshimura, Koji**
**4-11, Shinkofudai 4-chome,**
**Toyono-cho**
**Toyono-gun,**
**Osaka 563 01 (JP)**
Inventor: **Sasada, Reiko**
**C1-405, 2 Takenodai**
**Nagaokakyo,**
**Kyoto 617 (JP)**
Inventor: **Kaisho, Yoshihiko**
**332, Hamaderamotomachi 3-cho**
**Sakai,**
**Osaka 592 (JP)**
Inventor: **Iwane, Makoto**
**D73-104, 18 Tsukumodai 5-chome**
**Suita,**
**Osaka 565 (JP)**

**EP 0 414 151 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

(74) Representative: **von Kreisler, Alek, Dipl.-Chem. et al**
**Patentanwälte**
**von Kreisler-Selting-Werner**
**Postfach 10 22 41**
**D-50462 Köln (DE)**

## Description

### BACKGROUND OF THE INVENTION

The present invention relates to chinese hamster ovary (CHO) cells which are transformed with vectors containing human nerve growth factor (hereinafter also briefly referred to as NGF) genes and are cloned, and to methods for producing human NGF proteins which comprise cultivating the CHO cells in media.

NGF was discovered by Levi-Monntalcini [Ann. N.Y. Acad. Sci. 55, 330 (1952)] and Cohen et al. [Proc. Natl. Acad. Sci. U.S.A. 40, 1014 (1954)] and is a neurotrophic factor essential for the differentiation, growth and survival of peripheral nervous systems. Recently, it has been revealed that NGF has the action of maintaining the survival of cholinergic neurons in central nervous systems [Hefti, Journal of Neuroscience 6, 2155 (1986); Hatanaka et al., Dev. Brain Res. 39, 85 (1988)], and NGF is noted as a factor having a relation of some kind to Alzheimer's disease. Further, when NGF is given into the brains of aged rats, the improvement of memory disorder is observed [Nature 329, 65 (1989)]. From this fact, NGF is also expected as a therapeutic drug for senile dementia.

NGF (7S NGF) isolated from the submandibular glands of male mice is a complex ($\alpha_2\ \beta\ \gamma_2$)composed of three kinds of subunits $\alpha$, $\beta$ and $\gamma$, and NGF activity is observed only in the $\beta$ subunit of them. The $\beta$ subunit ($\beta$ NGF, 2.5S NGF) is a dimer of the same polypeptide consisting of 118 amino acids, and its amino acid sequence has been determined by Argeletti and Bradshaw [Proc. Natl. Acad. Sci. U.S.A. 68, 2417 (1971)].

Scott et al. succeeded in cloning a mouse $\beta$NGF gene from a mouse submandibular gland cDNA library by using as a probe an oligonucleotide which was synthesized on the basis of the amino acid sequence of mouse $\beta$NGF [Nature 302, 538 (1983)]. Furthermore, Ullrich et al. cloned an NGF gene from a human genome DNA library by using mouse $\beta$NGF cDNA as a probe, and proved that the amino acid sequence of human NGF deduced from its nucleotide sequence had 90% homology with that of mouse NGF [Nature 303, 821 (1983)].

The existence of human NGF has been indirectly proved by the above cloning of the human NGF gene, the detection of NGF mRNA in human brains [Gaedert et al., Molecular Brain Research 1, 85 (1986)] and the detection of NGF in human placentas and sperm using anti-mouse NGF antibodies [Heinrich and Meyer, Biochem. Biophys. Res. Commun. 155, 482 (1988)]. There have been no reports, however, that human NGF was isolated and that the protein chemical properties thereof were examined in detail. Galdstein et al. isolated NGF from human placentas and have reported that the molecular weight, isoelectric point and biological activities thereof were similar to those of mouse $\beta$NGF [Neurochemical Research 3, 175 (1978)]. However, the reproducibility of these results has not been recognized.

In order to prepare human NGF having such difficulty in isolation and purification, it is considered desirable to use genetic recombinant techniques. It has hitherto been reported that human NGF proteins were produced by Escherichia coli [S. Iwai et al., Chem. Pharm. Bull. 34, 4724 (1986); Itoh et al., Summaries of Lectures in the Annual Meeting of Pharmaceutical Society of Japan in 1988, p. 406 and EP-A-0 121 338]. In these cases, however, the recombinant human NGF proteins produced have very low activities, compared with that of mouse NGF. On the other hand, Kanaya et al. reported that a human NGF protein was secreted by yeast [Yeast Genetics and Molecular Biology News JAPAN 21, 35 (1988)]. However, its productivity is low (about 10 $\mu$g/l) and it is anticipated that only a part of the secreted proteins is active type.

Recently, Bruce and Heinrich expressed a human NGF gene in a COS cell and have reported that 15 ng/ml of active human NGF was produced in the medium [Neurobiology of Aging 10, 89 (1989)]. In this method, however, the COS cell is not cloned, so that a foreign gene is only temporarily expressed. For this reason, the foreign gene can not produce in large amounts using the COS cell.

### SUMMARY OF THE INVENTION

The present inventors tried to express human NGF genes using animal cell systems such as a CHO cell system which had the potential to stably express foreign genes, different from the above COS cell system. As a result, the present inventors discovered that a significant amount of human NGF could be produced by cultivating in media animal cells which were transformed with vectors containing genes coding for human NGF and were cloned, and further that active human NGF proteins containing specific disulfide bonds could be obtained by purifying a significant amount of human NGF thus produced. The present inventors further conducted investigations based on this finding, and completed the present invention.

The present invention provides:

(1) a Chinese hamster ovary (CHO)cell which is transformed with a vector containing a gene coding for a human NGF protein and is cloned, and

(2) a method for producing a human NGF protein which comprises cultivating the above CHO cell.

## BRIEF DESCRIPTION OF THE DRAWINGS

Fig. 1 shows a nucleotide sequence of a cloned human NGF gene obtained in Reference Example 1 and an amino acid sequence translated therefrom;

Fig. 2 is a schematic representation showing the construction of human NGF expression vector pMNGF101 obtained in Reference Example 1;

Fig. 3 is a schematic representation showing the construction of human NGF expression vector pMNGF201 obtained in Reference Example 2;

Fig. 4 is a schematic representation showing the construction of human NGF expression vector pTB1058 obtained in Example 1;

Fig. 5 shows SDS-polyacrylamide gel electrophoresis diagrams of purified human NGF and mouse NGF obtained in Example 5, in which the electrophoresis was carried out in accordance with the method of Laemmli under reduction conditions, and (1) and (2) show diagrams for the standard sample of mouse 2.5SNGF and for purified human NGF, respectively;

Fig. 6 is a graph showing the PC12 cell neurite outgrowth activity of purified human NGF obtained in Example 5, in which PC12 cells were cultivated in the presence of human NGF (●) or mouse 2.5SNGF (o) for 2 days and the ratio (%) of cells having neurites was calculated; and

Fig. 7 is an analytical representation showing the disulfide bonds form of purified human NGF obtained in

Example 11.

## DESCRIPTION OF THE PREFERRED EMBODIMENTS

The human NGF proteins to be produced according to the present invention include human NGF and muteins thereof. The human NGF includes human NGF derived from natural products such as placenta derived-human NGF, synthetic human NGF and human NGF produced by genetic engineering techniques. As the human NGF, human NGF having 120 amino acid residues which is obtained in Examples described below is preferable.

The muteins of the human NGF includes muteins obtained by mutagenizing the amino acid sequence of the original peptide or protein, other than 6 cysteine residues which are bound each other by disulfide bonds in its molecule. Such mutagenesis includes addition of an amino acid(s), deletion of a constituent amino acid(s) and substitution of a constituent amino acid(s) for another amino acid(s). Therefore, the mutein contains 6 cysteine residues in the above molecule.

Such addition of an amino acid(s) includes addition of at least one amino acid.

Such deletion of a constituent amino acid(s) includes deletion of at least one human NGF-constituent amino acid.

Such substitution of a constituent amino acid(s) for another amino acid(s) includes substitution of at least one human NGF-constituent amino acid for another amino acid(s).

At least one amino acid in the mutein which has at least one amino acid added to the human NGF excludes methionine caused by an initiation codon used for expression of a peptide, or a signal peptide.

The number of added amino acid residues is at least one, but it may be any number as long as the characteristics of the human NGF are not lost. A portion or the whole of the amino acid sequence of a protein having homology with the human NGF and exhibiting activities similar to those of the human NGF is more preferably used.

As to the number of deleted constituent amino acids in the mutein which lacks at least one human NGF-constituent amino acid, it may be any number as long as the characteristics of the human NGF are not lost.

As to the number of at least one human NGF-constituent amino acids before substitution in the mutein which has at least one human NGF-constituent amino acid substituted for another amino acid(s), it may be any number as long as the characteristics of the human NGF are not lost.

Examples of the constituent amino acids before substitution include amino acids other than cysteine. The amino acids other than cysteine as the constituent amino acids before substitution include aspartic acid, arginine, glycine, serine and valine.

As the other substituted amino acids, for example, amino acids different in hydrophilicity, hydrophobicity or electric charge from the constituent amino acid before substitution are selected.

Specifically, when the amino acid before substitution is aspartic acid, the substituting amino acids include asparagine, threonine, valine, phenylalanine and arginine, and particularly, asparagine and arginine are preferable.

When the amino acid before substitution is arginine, the substituting amino acids include glutamine, threonine, leucine, phenylalanine and aspartic acid, and particularly, glutamine is preferable.

When the constituent amino acid before substitution is glycine, the substituting amino acids include threonine, leucine, phenylalanine, serine, glutamic acid and arginine, and particularly, threonine is preferable.

When the constituent amino acid before substitution is serine, the substituting amino acids include methionine, alanine, leucine, cysteine, glutamine, arginine and aspartic acid, and particularly, methionine is preferable.

When the constituent amino acid before substitution is serine, the substituting amino acids include methionine, alanine, leucine, cysteine, glutamine, arginine and aspartic acid, and particularly, methionine is preferable.

When the constituent amino acid before substitution is valine, the substituting amino acids include serine, leucine, proline, glycine, lysine and aspartic acid, and particularly, serine is preferable.

As the substituting amino acids, asparagine, glutamine, arginine, threonine, methionine, serine and leucine are preferred.

In the above substitution, two or more substitutions may be carried out at the same time.

The above muteins may be mutagenized by a combination of 2 or 3 of the above addition, deletion and substitution.

In order to produce the above muteins, site-directed mutagenesis is employed. Site directed mutagenesis, a well known procedure, is disclosed in R. F. Lather and J. P. Lecoq, Genetic Engineering, Academic Press, p.31-50 (1983). Mutagenesis directed to oligonucleotide is described in M. Smith and S. Gillam, Genetic Engineering: Principles and Methods, Plenum Press, vol.3, p.1-32 (1981).

A structural gene coding for the above mutein is produced, for example, by the steps of:

(a) hybridizing a single-stranded DNA consisting of one strand of a structural gene of the human NGF with a mutagenic oligonucleotide primer (the primer is complementary to a codon of an amino acid to be substituted for this single-stranded DNA or to a region containing an anti-sense triplet which pairs to this codon in some cases, provided that the same shall not apply to discrepancy of this codon from other codons coding for the amino acids or the anti-sense triplet in some cases).

(b) elongating the primer with DNA polymerase to form a mutational heteroduplex, inserting the mutational heteroduplex into double stranded phage DNA, and transfecting the phage DNA to E. coli, and

(c) replicating this mutational heteroduplex in E. coli.

Then, the phage DNA carrying the mutational gene is isolated, and incorporated into a plasmid.

A suitable host is transformed with the plasmid thus obtained, and the resulting transformant is cultivated in a medium, whereby the mutein can be produced.

The genes coding for the human NGF proteins of the present invention include, for example, genes obtained from human genome libraries by cloning and genes obtained by chemical synthesis. The cloning of the genes coding for the human NGF proteins can be carried out, for example, by the method described in Nature 303, 821 (1983).

The thus obtained gene coding for the human NGF protein can be used as it is or cut out with a restriction enzyme, depending on the intended use.

The gene coding for the human NGF protein obtained above is ligated downstream from a promoter. In this case, it is desirable to ligate a DNA coding for a signal peptide, or a DNA coding for the signal peptide and a DNA coding for a propetide, to the 5′-terminus of the gene coding for the human NGF protein. Any peptide can be used as the signal peptide as long as it can secrete the human NGF protein. Specific examples of such signal peptides include signal peptides of human, mouse, rat, bovine and chicken NGFs, a signal peptide of egg-white lysozyme and mutants thereof, and a signal peptide of human interleukin-2. Further, the propetides include propeptides of human, rat, mouse, bovine and chicken NGFs.

In addition to the above methods, the human NGF protein can also be obtained by producing a fused protein of the human NGF protein and another protein by secretion and then cleaving it with an appropriate protease.

The human NGF protein expression vectors for CHO cells are constructed using the above DNA coding for the human NGF protein.

Examples of vectors used for construction of the human NGF protein expression vectors include pBR322 and derivatives thereof, SV40-series vectors, bovine papilloma virus vectors, retrovirus vectors and BK virus vectors. In addition, animal viruses such as EB virus and herpes simplex virus can also be used as the vectors.

As the promoter used for the expression vector, any promoter is available as long as it is functionable in animal cells. Examples of such promoters include an SV40 promoter, an LTR promoter and a metallothionein promoter.

In addition to the above, enhancers, RNA splicing signals, poly A addition signals or selected markers are used for the expression vectors.

Methods for constructing expression vectors are known per se and described, for example, in Molecular Cloning, A Laboratory Manual, Cold Spring Harbor Laboratory (1982).

The animal cells are transformed with the human NGF protein expression vectors thus prepared.

Methods for transforming the CHO cells are known in the art, and include, for example, the method of Graham et al. [Virology 52, 456 (1973)].

As described above, the CHO cells transformed with the human NGF protein expression vectors are obtained.

Methods for stably expressing the human NGF protein genes using the animal cells transformed with the above human NGF protein expression vectors include methods in which the human NGF protein expression vectors are incorporated into chromosomes of introduced cells, and methods in which the human NGF protein expression vectors stably exist without incorporation thereof in chromosomes of introduced cells. In the former case, the production of the human NGF proteins can be increased using amplifier systems such as a dihydrofolate reductase (DHFR) gene [J. Mol. Biol. 159, 601 (1982)].

Methods for selecting the transformed CHO cells (clones) are known per se in the art, and described, for example, in Experimental Medicine (Japanese) (Yoshisha), Extra Number, Vol. 5, No. 11 (1987). Specifically, the transformant strain is selected with the guidance of a selected marker gene together with the human NGF protein gene. In this case, the selected marker may be placed on the vector having the human NGF protein gene to introduce it into the cell, or the selected marker may be co-transfected into the cell together with a larger amount of human NGF Protein genes without introduction into the same vector. Such selected markers include, for example, dihydrofolate reductase (DHFR) [methotrexate (MTX) resistance], thymidine kinase, an Ecogpt gene (mycophenolic acid resistance) and neo gene (G418 resistance). The transformant strains thus obtained using the selected markers are further repeatedly subjected to clone selection, whereby the stable cell strains having the high productivity of gene products can be obtained.

When the CHO cells thus obtained are cultivated, examples of media include MEM containing about 0.5 to 20% fetal calf serum [Science 122, 501 (1952)], DMEM [Virology 8, 396 (1959)], RPMI1640 medium [J. Am. Med. Assoc. 199, 519 (1967)] and 199 medium [Proc. Soc. Exp. Biol. Med. 73, 1 (1950)]. The pH is preferably 6 to 8. The cultivation is usually carried out at 30 to 40 °C for 15 to 60 hours, with aeration or agitation if necessary.

The human NGF proteins of the present invention are produced and accumulated inside or outside the cells. When the intracellular human NGF proteins are extracted from the cultivated cells, the cells are collected by methods known in the art after cultivation. Then, the collected cells are suspended in an appropriate buffer solution containing a protein denaturant such as guanidine hydrochloride or urea, or a buffer solution containing a surface-active agent such as Triton X-100, followed by centrifugation to obtain a supernatant containing the human NGF protein. There are also suitably used methods in which the cells are disrupted by ultrasonic treatment or freeze-thawing, followed by centrifugation to obtain a supernatant containing the human NGF protein.

The separation and purification of the human NGF proteins contained in the supernatants or produced and accumulated outside the cells can be carried out by suitable combinations of separating and purifying methods known per se in the art.

These well known separating and purifying methods include methods utilizing a difference in solubility such as salt precipitation, ammonium sulfate precipitation and solvent precipitation; methods mainly utilizing a difference in molecular weight such as dialysis, ultrafiltration and SDS-polyacrylamide gel electrophoresis; methods utilizing a difference in electric charge such as ion-exchange chromatography; methods utilizing specific affinity such as affinity chromatography; methods utilizing a difference in hydrophobicity such as reverse-phase high performance liquid chromatography (HPLC); methods utilizing a difference in specific affinity such as antibody columns and metal chelate columns (for example, $Cu^{2+}$ column) ; and methods utilizing a difference in isoelectric point such as chromatofocusing.

The human NGF protein having a purity of 90% (w/w) or more, preferably of 94% (w/w) or more, is thus obtained as an active form. The purity is assayed from HPLC, SDS-PAGE or biological activity. Thus, the purity of the human NGF protein is preferably 94% or more.

The human NGF protein of the present invention thus obtained is an active form, and has 6 cysteine residues in its molecule, in which the first cysteine residue from the N-terminus is bound to the fourth cysteine residue, the second cysteine residue is bound to the fifth cysteine residue, and the third cysteine residue is bound to the sixth cysteine residue to form disulfide bonds, therebetween.

The human NGF proteins obtained as described above are assayed by immunoassays or methods based on the biological activities.

Examples of the former immunoassays include the enzyme immunoassay (EIA) described in Biochem. Biophys. Res. Commun. 155, 482 (1988). Examples of the latter methods include methods for assaying the biological activities with the guidance of the outgrowth of nerve fibers in ganglions of chicken embryonic dorsal spinal roots (Cell Growth Factors, edited by Tissue Culture Society of Japan, Asakura Shoten, 1984) or in rat adrenal medulla derived-PC12 cells [Brain Research 133, 350 (1977)], or with guidance of the induction of choline acetyltransferase activity in rat septal area cholinergic neurons.

The human NGF proteins thus obtained are useful as reagents for the studies of brains and nerves, and can also be expected as therapeutic drugs for senile dementia.

When the human NGF protein is used for these studies, it is preferred to add the human NGF protein to the medium for cultivation of animal cells to give a final concentration of 0.1 to 1,000 ng/ml of medium, more preferably 1 to 100 ng/ml of medium.

The human NGF proteins having specific disulfide bonds can be advantageously used as reagents or therapeutic drugs, because of their active forms.

Since the CHO cells of the present invention have been cloned, introduced genes can be stably expressed. Accordingly, when the cloned CHO cells of the present invention are used, the high pure human NGF proteins can be commercially produced in large amounts.

When bases, amino acids and so on are indicated by the abbreviations in this specification and the drawings, the abbreviations adopted by IUPAC-IUB Commission on Biochemical Nomenclature or commonly used in the art are employed. For example, the following abbreviations are used. When the optical isomers are capable of existing with respect to the amino acids, the L-forms are represented unless otherwise specified.

DNA : Deoxyribonucleic acid
A : Adenine
C : Cytosine
G : Guanine
T : Thymine
A or Ala : Alanine
R or Arg : Arginine
N or Asn : Asparagine
D or Asp : Aspartic acid
C or Cys : Cysteine
Q or Gln : Glutamine
E or Glu : Glutamic acid
G or Gly : Glycine
H or His : Histidine
I or Ile : Isoleucine
L or Leu : Leucine
K or Lys : Lysine
M or Met : Methionine
F or Phe : Phenylalanine
P or Pro : Proline
S or Ser : Serine
T or Thr : Threonine
W or Trp : Tryptophan
Y or Tyr : Tyrosine
V or Val : Valine

The present invention will hereinafter be described in detail with the following Reference Examples and Examples. It is understood of course that these are not intended to limit the scope of the invention.

Escherichia coli DH1/pMNGF101 obtained in Reference Example 1 was deposited with the Institute for Fermentation, Osaka, Japan (IFO) under the accession number IFO 14869 on April 6, 1989. This microorganism was also deposited with the Fermentation Research Institute, Agency of Industrial Science and Technology, Ministry of International Trade and Industry, Japan (FRI) under the accession number FERM BP-2385 on April 15, 1989. Escherichia coli DH1/pMNGF201 obtained in Reference Example 2 was deposited with the IFO under the accession number IFO 14870 on April 6, 1989. This microorganism was also deposited with the FRI under the accession number FERM BP-2386 on April 15, 1989.

Transformant CHO cell (cloned)CHO-D5 obtained in Example 3 was deposited with the IFO under the accession number IFO 50195 on July 17, 1989. This cell was also deposited with the FRI under the accession number FERM BP-2544 on August 4, 1989. Transformant CHO cell (cloned) CHO-D31-10 obtained in Example 4 was deposited with the IFO under the accession number IFO 50217 on November 15, 1989. This cell was also deposited with the FRI under the accession number FERM BP-2674 on December 7, 1989.

Transformant CHO cell (cloned) CHO-D31-10-2 obtained in Example 7 was deposited with the IFO under the accession number IFO 50236 on March 21, 1990. This cell was also deposited with the FRI under the accession number FERM BP-2851 on April 6, 1990.

The above FERM numbers and BP numbers show the accession numbers under the Budapest treaty.

## Reference Example 1

### Construction of Human NGF Expression Vector (1)

Escherichia coli NM538 was infected with A λEMBL3 genomic library (Clontech) prepared from human leukocyte DNA, and about 3 X $10^4$ clones thereof were spread on each soft agar plate. The plaques were transferred on nylon membranes (Hybond-N, Amersham), and then immersed in a 0.5 N NaOH- 1.5 M NaCl solution for 6 minutes to denature phage DNA, followed by immersion in a 0.5 M Tris-HCl(pH 8.0)-1.5 M NaCl solution for 6 minutes. The membranes were immersed in a 2 X SSC solution, and then air-dried, followed by treatment at 80°C for 2 hours to fix the DNA on the membranes.

On the other hand, a 0.38-kb DNA fragment coding for human βNGF was chemically synthesized with reference to the known human NGF gene [A. Ullrich et al., Nature 303, 821 (1983)], and labeled with $^{32}P$ using a DNA labeling kit (Nippon Gene) to form a probe.

The filters on which the DNA was fixed were maintained at 65°C for 16 hours in 10 ml of a solution containing 6 X SSC (1 X SSC = 0.15 M NaCl, 0.015 M sodium citrate), 5 X Denhardt's, 0.5% SDS, 20 µg/ml of denatured salmon sperm DNA and the labeled probe. After completion of the reaction, the filters were washed with a solution (2 X SSC, 0.1% SDS) at room temperature for 5 minutes 3 times and further washed with a solution (1 X SSC, 0.1% SDS) at 60°C for 60 minutes. After the washed filters were dried, radioautograms were taken, and the clones reactive to the probe were searched. From clone βLN2113 obtained by this method, the phage DNA was extracted according to the method of Davis et al. [Davis et al., Advanced Bacterial Genetics, Cold Spring Harbor Laboratory (1980)].

Then, λ βLN2113 was cleaved with SmaI and ApaI to cut out a DNA fragment of about 1 kb containing a human NGF gene, and it was inserted into the SmaI and ApaI sites of plasmid p Bluescript IIK (purchased from Toyobo) to obtain plasmid pNGFP107G. The nucleotide sequence of the inserted portion was determined by using Seeknase (Biochemical) (Fig. 1). The determined nucleotide sequence was completely identified with the sequence described in Nature 303, 821 (1983) in the protein coding region.

The above phage λ βLN2113 DNA was cleaved with restriction enzyme BglII to isolate a 1.8-kb DNA fragment containing human NGF. On the other hand, expression vector pKSV-10 for animal cells (Pharmacia) was cleaved with restriction enzyme BglII, and the resulting fragment was ligated to the above 1.8-kb DNA fragment containing the human NGF gene with T4 DNA ligase. Escherichia coli DH1 was transformed using this reaction solution. A plasmid isolated from Escherichia coli DH1/pMNGF101 (IFO 14869, FERM BP-2385), one of ampicillin-resistant transformants, was named pMNGF101 (Fig. 2).

## Reference Example 2

### Construction of Human NGF Expression Vector (2)

The plasmid pNGFP107G obtained in Reference Example 1 was cleaved with restriction enzymes BclI and ApaI to isolate 0.8-kb DNA fragment containing the human NGF gene. The resulting 0.8-kb BclI-ApaI fragment was mixed with chemically synthetic adaptors SN1, SN2 and SN3 (refer to Fig. 3), and ligated thereto with T4 DNA ligase, followed by cleaving with BglII to obtain a 0.8-kb HindIII-BglII DNA fragment.

Plasmid pSV2-gpt [Science 209 1422 (1980)] was cleaved with restriction enzymes EcoRI and HindIII to isolate a 2.6-kb EcoRI-HindIII DNA fragment containing an SV40 promoter. Then, a 1.6-kb BglII-EcoRI fragment containing a poly A addition region was isolated from plasmid pMTVdhfr [Nature 294, 228 (1981)].

The above 2.6-kb EcoRI-HindIII fragment containing the SV40 promoter, the above 0.8-kb HindIII-BglII DNA fragment containing the human NGF gene and the above 1.6-kb BglII-EcoRI fragment containing the poly A addition region were ligated to one another with T4 DNA ligase. Escherichia coli DH1 was transformed using this reaction solution. A plasmid isolated from an ampicillin-resistant transformant [Escherichia coli DH1/pMNGF201 (IFO 14870, FERM BP-2386)] was named pMNGF201 (Fig. 3).

Reference Example 3

(1) Synthesis of Human NGF N-Terminal Peptide, H-Ser Ser Ser His Pro Ile Phe His Arg Gly Glu Phe Ser Val Cys-OH

The human NGF N-terminal peptide was synthesized by a solid-phase method using an automatic peptide synthesizer 430A (Applied Biosystems). As a program, "Standard" was used. The synthesis was basically conducted in accordance with the method described in R. B. Merrifield, Adv. Enzymol. 32, 221-296 (1969). Boc-Cys(MeBzl)*PAM-P (0.5 mmole/g) was used as a resin, and the synthesis was carried out sequentially from the carboxyl terminus. As Boc-amino acids, there were used Boc-Val, Boc-Ser(Bzl), Boc-Phe, Boc-Glu(OBzl), Boc-Gly, Boc-Arg(Tos), Boc-His(Tos), Boc-Ile and Boc-Pro. After synthesis up to the amino terminus Ser, the peptide resin was taken out of the synthesizer and dried.

To 1 g of the peptide resin were added 1.5 ml of p-cresol and 0.5 ml of 1,2-ethanedithiol, and about 8 ml of liquid hydrogen fluoride was further added thereto, followed by reaction at 0°C for 2 hours. After completion of the reaction, hydrogen fluoride was removed under reduced pressure in a desiccator, and washed with a 0.1% solution of 2-mercaptoethanol in diethyl ether, followed by washing with diethyl ether to remove most of the included reagents. The peptide was extracted with 10 ml of 3% acetic acid, and the resin included in the extracted solution was removed by filtration. The filtrate was purified by gel permeation chromatography using a Sephadex G-25 column. The conditions of the gel permeation chromatography were as follows:

Column size: 2.8 X 60 cm; Detecting wavelength: 230 nm;
Solvent: 3% acetic acid; Flow rate: 40 ml/hr

Fractions containing the peptide were collected and lyophilized to obtain a powdery sample. The resulting powdery sample was further purified by reverse-phase high performance liquid chromatography under the following conditions:

Column: YMC pack, A-324 ODS 10 X 250 mm;
Column temperature: 25°C;
Eluent A: 0.1% trifluoroacetic acid-99.9% distilled water;
Eluent B: 0.1% trifluoroacetic acid-99.9% acetonitrile;
Elution program: 0 minute (90% A + 10% B), 30 minutes (60% A + 40% B)
Elution rate: 1.6 ml/min;
Detecting wavelength: 230 nm

Main peak fractions eluted at a retention time of 29.14 minutes under these conditions were collected and passed through a Bio RAD AGI X 8 column (AcOH type, 1.8 X 5 cm). Washings were also collected. After acetonitrile was removed by distillation, lyophilization was carried out. Thus, 76 mg of white powder was obtained.

Thin layer chromatography: Rf = 0.71 (Avicel gel plate, developing solvent; n-butanol:AcOH:pyridine:water = 30:20:6:4)

Assay of free SH groups by the method described in G. L. Elman, Arch. Biochem. Biophys. 82, 70-77 (1959): 97%

Anal. for amino acids: Ser 3.99(4); Glu 1.00(1); Pro 0.98(1); Gly 1.19(1); 1/2Cys 0.84(1); Val 1.03(1); Ile 1.01(1); Phe 2.12(2); His 1.84(2); Arg 0.94(1)

Recovery: 71.1%

1/2Cys was assayed by the performic acid oxidation method. The values in parentheses show theoretical values.

(2) Preparation of Conjugate of Human NGF N-Terminal Peptide and Bovine Serum Albumin

Bovine serum albumin (BSA) (132 mg) was dissolved in 3 ml of 0.1 M phosphate buffer (pH 7.5). A dimethylamide solution (200 µl) containing 11.2 mg of N-(γ-maleimide-butyloxy)succinimide (GMBS) was added dropwise to the above solution while stirring with a stirrer, and the mixture was reacted at 30°C for 30 minutes. The reaction solution was purified by a Sephadex G-25 (1.5 X 30 cm) using 0.1 M phosphate buffer (pH 6.5)-0.1 M NaCl as an eluent to obtain BSA into which maleimide groups were introduced (maleimidated BSA).

The above maleimidated BSA (20 mg) was dissolved in 0.1 M phosphate buffer (pH 6.5)-0.1 M NaCl. On the other hand, the human NGF N-terminal peptide (5 mg) obtained in (1) was dissolved in 0.1 M phosphate buffer (pH 6.0)-5 mM EDTA. Both of the solutions were mixed with each other (the total volume is 5 ml or less), followed by reaction at 30 °C for 60 minutes. Then, PBS was added thereto to obtain 12 ml of a solution containing a conjugate of the human NGF N-terminal peptide and BSA. 1.5 ml parts of this solution were used for immunization of the rabbits.

(3) Preparation of Anti-Human NGF N-Terminal Peptide Antibody

The conjugate of the human NGF N-terminal peptide and BSA obtained above was thoroughly mixed with Freund's complete adjuvant, and the resulting mixture was subcutaneously injected into the rabbits. Thereafter, a mixture of the above conjugate and Freund's incomplete adjuvant was injected into the same rabbits at 2-week intervals.

Blood collected from the rabbits immunized as described above was centrifuged to obtain an antiserum. The antibody titer of the resulting antiserum was assayed by an ELISA using a conjugate of the human NGF N-terminal peptide previously described and human serum albumin as an antigen. As a result, a high antibody titer was observed.

The above antiserum was purified by ammonium sulfate precipitation and DEAE cellulose column chromatography to obtain an anti-human NGF N-terminal peptide antibody.

Example 1

Construction of Human NGF Expression Vector

Plasmid pMNGF201 obtained in Reference Example 2 was cleaved with HindIII, and the cleavage end was rendered flush by the DNA polymerase Klenow fragment reaction, followed by cleavage with BglII to isolate a DNA fragment of about 0.8 kb. On the other hand, plasmid pTB399 (described in Japanese Patent Unexamined Publication No. 61- 63282/1986 corresponding to EP-172,619) was cleaved with EcoRI, and the cleavage end was rendered flush by the Klenow fragment reaction, followed by cleavage with BglII to obtain a DNA fragment of about 3.9 kb. Both of these DNA fragments were linked and cyclized by T4 DNA ligase reaction to obtain plasmid pTB1054.

Then, plasmid pTB348 (described in Japanese Patent Unexamined Publication No. 61-63282/1986 corresponding to EP-172,619) having hamster DHFR cDNA was cleaved with ClaI, followed by treatment with an alkaline phosphatase. The resulting fragment was mixed with a 2.4-kb DNA fragment [containing MnLVLTR, a human NGF hNGF) gene, an SV40 DNA-derived splicing region and a poly A addition region] which was separated and purified from the plasmid pTB1054 cleaved with ClaI to construct human NGF expression vector pTB1058 by the T4 DNA ligase reaction (Fig. 4).

Example 2

Transformation of CHO Cell

Ham 12 medium containing 5% fetal calf serum was placed in a Falcon dish (6 cm in diameter), and hamster $DHFR^-$CHO cells were cultivated therein at 37 °C overnight. After cultivation, the cells (7 x $10^5$ cells/dish) were transformed in accordance with the method of Graham et al. [Virology 52, 456-467 (1973)] using 10 µg of the human NGF expression vector pTB1058 obtained in Example 1. After cultivation at 37 °C for 4 hours, the medium was exchanged for new one, and cultivation was continued. After 2 days from the exchange, the medium was exchanged for Dulbecco's modified MEM containing 5% dialyzed fetal calf serum and 35 µg/ml of proline. After that time, cultivation was continued in this selective medium. After about 2 to 3 weeks, the cells which proliferated as $DMFR^+$ formed colonies.

Example 3

Cloning of Transformant and Expression of Human NGF Gene

The transformant cells obtained in Example 2 were cloned in accordance with methods known in the art (for example, limited dilution methods) to obtain transformants (cloned) CHO-D5 (IFO 50195, FERM BP-2544), CHO-D42 and CHO-M36.

After completion of the cloning, the clonal cells of each transformant were cultivated in Dulbecco's modified MEM containing 5% fetal calf serum, 35 µg/ml of proline, 50 IU/ml of penicillin and 50 µg/ml of streptomycin. The separated clonal cells of each transformant were inoculated on a Limbro dish. When about 80% of the cells became confluent, the medium was exchanged for new one. After cultivation for 72 hours, the NGF in the culture supernatant was determined by the EIA [Behringer; Biochem. Biophys. Res. Commun. 155, 482 (1988)].

Table 1 shows clones which were high in production of the human NGF. No NGF was detected in the culture supernatant of CHO cells not transformed.

Table 1

| Transformant (clone) | NGF (ng/ml) |
|---|---|
| CHO-D5 | 110 |
| CHO-D42 | 39 |
| CHO-M36 | 24 |

The above results reveal that the CHO cell which permanently expresses the human NGF gene can produce a larger amount of human NGF than the COS cell which temporarily expresses the gene.

Example 4

CHO Cell Strain High in Production of Human NGF

Transformant CHO-D31 obtained in a manner similar to that of Example 3 was cultivated in Dulbecco's modified MEM (containing 5% fetal calf serum and 35 µg/ml of proline) containing 10 nM methotrexate (MTX). Since the clones of this transformant showed normal proliferation at this concentration of MTX, the MTX concentration was increased to 100 nM and subculture was continued. When the MTX concentration was further increased to 1 µM, most of the cells died. However, when the medium was exchanged for new one after 3 or 4 days and cultivation was continued, several cells per $10^5$ cells began to proliferate in a colony form. After full proliferation of these cells, the cells were subcultured in a culture solution of 10 µM MTX. Thereupon, most of the cells died again, and several cells began to proliferate in a colony form. The cells thus obtained showed stable, normal proliferation in the presence of 10 µM · MTX, and normally proliferated even when the cells were cultivated in the presence of 10 µM MTX after they had been subcultured through several cycles in a culture solution free from MTX. The 10 µM MTX resistant CHO-D31-10 cells (IFO 50217, FERM BP-2674) thus obtained were cultivated under the same conditions as with Example 3. As a result, it was proved by the EIA that $4X10^3$ ng/ml of human NGF was produced in the medium.

Example 5

Isolation of Human NGF (1)

Cell strain CHO-D31-10 obtained in Example 4 was cultivated in large amounts in Dulbecco's modified medium containing 5% fetal calf serum, 35 µg/ml of proline, 50 IU/ml of penicillin, 50 µg/ml of streptomycin and 10 µM methotrexate under an atmosphere of 5% carbon dioxide at 37°C for 7 days. Consequently, it was proved by the EIA that 2.4 mg/l of human NGF was produced in the medium.

The culture solution was centrifuged, and APMSF was added to 2.2 liter of the resulting culture supernatant to a final concentration of 0.1 mM. The solution thus obtained was adjusted to pH 6.0 with 0.2 N acetic acid, followed by centrifugation. The supernatant thus obtained was adsorbed into an S-Sepharose column (2.6 cm X 14 cm) equilibrated with 0.1 M phosphate buffer(pH 6.0)-1 mM EDTA, and washed with 0.1 M phosphate buffer(pH 6.0)-0.15 M NaCl-1 mM EDTA-10% glycerol, followed by elution with 50 mM Tris-HCl(pH 7.5)-0.7 M NaCl-1 mM EDTA-10% glycerol. The fractions containing the human NGF were collected and concentrated about 30-fold with a Diaflow cell (Type YM10, Amicon). The resulting concentrated solution was purified by gel permeation chromatography with a Sephacryl S-100HR column (200 ml, 1.6 cm X 100 cm) equilibrated with 20 mM Tris-HCl(pH 7.4)-0.5 M NaCl-1 mM EDTA-10% glycerol. The fractions containing the human NGF were collected and concentrated about 10-fold on a Centriprep 10 (Amicon). The resulting concentrated solution was subjected to a reverse-phase HPLC to purify the human NGF. Namely, the concentrated solution was applied on Asahipak ODP-50 column (10.0 mm ID X 250 mm L), which was eluted with a linear gradient of 0 to 90% acetonitrile containing 0.1% trifluoroacetic acid to obtain 1.2 mg (from amino acid analysis) of a purified human NGF sample. Table 2 shows the summary of this purification. As a result of SDS-polyacrylamide gel electrophoresis (Fig. 5) and reverse-phase HPLC, the purity of the resulting recombinant human NGF was 94%.

Table 2

| Summary of Purification of Recombinant Human NGF | | | | |
|---|---|---|---|---|
| | Solution volume (%) | Total protein (mg) | Total Human NGF (mg) | Yield (%) |
| Culture supernatant | 2,200 | 11,000 | 5.3 | 100 |
| S-Sepharose | 4.5 | 24 | 5.0 | 94 |
| Sephacryl S-100 | 2.5 | 3.7 | 4.7 | 89 |
| Reverse-phase HPLC | | 1.3 | 1.6 | 30 |

The purified sample thus obtained was subjected to 16% polyacrylamide gel electrophoresis containing 0.1% SDS, and then detected by silver staining. As a consequence, a single band was observed at the position corresponding to a molecular weight of 13,000 (Fig. 5). Also in Western blot analysis using the rabbit anti-human NGF N-terminal peptide antibody obtained from rabbit serum immunized by a synthetic peptide consisting of amino terminal 15 amino acid residues of human NGF (refer to Reference Example 3) and affinity-purified HRP-linked goat anti-rabbit IgG as a second antibody(Bio-Rad, U.S.A.), a band was observed at the same position as described above.

The purified human NGF thus obtained was taken in a glass test tube for hydrolysis, and dried under reduced pressure. Then, after 5.7 N hydrochloric acid containing 4% thioglycolic acid was added thereto, the test tube was sealed under reduced pressure, followed by hydrolysis at 110°C for 24 hours. After hydrolysis, hydrochloric acid was removed and the residue was dissolved in 0.02 N hydrochloric acid to carry out amino acid analysis.

The results are shown in Table 3.

Table 3

| Amino Acid Composition | | |
|---|---|---|
| Amino acid | Experimental 1) value | Theoretical 2) value |
| Asp + Asn | 13.0 | 13 |
| Thr | 9.7 | 10 |
| Ser | 9.2 | 11 |
| Glu + Gln | 6.6 | 6 |
| Pro | 2.9 | 3 |
| Gly | 7.3 | 7 |
| Ala | 6.9 | 7 |
| Val | 12.8 | 13 |
| Met | 2.1 | 2 |
| Ile | 6.1 | 6 |
| Leu | 3.2 | 3 |
| Tyr | 2.3 | 2 |
| Phe | 7.3 | 7 |
| Lys | 9.1 | 9 |
| His | 3.9 | 4 |
| Arg | 7.3 | 8 |
| Trp | 3.0 | 3 |
| Total | | 120 |

1) Calculated taking Asp + Asn as 13.
2) Calculated from the amino acid sequence deduced from the nucleotide sequence of the human NGF gene.

On the other hand, Ullrich et al. estimate that human NGF consists of 118 amino acids, from the comparison with mouse βNGF [Nature 303, 821 (1983)].

The N-terminal amino acid sequence of the purified human NGF was determined by using a gas-phase protein sequencer (Model 470A, Applied Biosystems). The results are shown in Table 4.

Table 4

| N-Terminal Amino Acid Sequence | | | |
|---|---|---|---|
| Cycle | PTH-amino acid | | Amino acid sequence deduced from the DNA sequence |
| | Residue | pmole | |
| 1 | Ser | 605 | Ser |
| 2 | Ser | 495 | Ser |
| 3 | Ser | 384 | Ser |
| 4 | His | 785 | His |
| 5 | Pro | 705 | Pro |
| 6 | Ile | 767 | Ile |
| 7 | Phe | 829 | Phe |
| 8 | His | 341 | His |
| 9 | Arg | 700 | Arg |
| 10 | Gly | 425 | Gly |
| 11 | Glu | 478 | Glu |
| 12 | Phe | 517 | Phe |
| 13 | Ser | 97 | Ser |
| 14 | Val | 467 | Val |
| 15 | - | | Cys |
| 16 | Asp | 297 | Asp |
| 17 | Ser | 58 | Ser |
| 18 | Val | 392 | Val |
| 19 | Ser | 73 | Ser |
| 20 | Val | 476 | Val |
| 21 | Trp | 95 | Trp |
| 22 | - | | Val |
| 23 | Gly | 166 | Gly |
| 24 | Asp | 179 | Asp |
| 25 | Lys | 245 | Lys |
| 26 | Thr | 74 | Thr |
| 27 | Thr | 102 | Thr |
| 28 | Ala | 179 | Ala |

2.9 nmoles of the purified human NGF was used for analysis.

The C-terminal amino acid of the purified human NGF which was examined by hydrazinoiysis [Natita et al., J. Biochem. 59, 170 (1966)] was alanine. Accordingly, it became clear that the above human NGF consisted of 120 amino acids.

As a result of examination by PAG isoelectro focusing electrophoresis [New Experimental Methods of Electrophoresis, edited by Electrophoresis Society, Bunkodo (1989)], the isoelectric point of the resulting human NGF was pH 9 to 10, and similar to that of mouse NGF (Collaborative Research Inc.).

In accordance with the method described in Brain Research 133, 350 (1977), Experimental Cell Research 145, 179 (1983) and Journal of Neuroscience Research 17, 25 (1987), the activity of the purified human NGF was assayed with the guidance of the neurite outgrowth of cell PC12. As a result, the purified human NGF exhibited an activity similar to that of the standard sample of mouse 2.5S-NGF (Wako Pure Chemical Industries, Japan) (Fig. 6).

In accordance with the method described in Developmental Biology 111, 62 (1985), the function of the purified human NGF to avian embryo dorsal root ganglia (DRG) was examined. The results showed that the purified human NGF promoted the neurite outgrowth and survival of DRG derived-nerve cells.

Example 6

Isolation of Human NGF (2)

Cell strain CHO-D31-10 obtained in Example 4 was cultivated in a manner similar to that of Example 3. Consequently, it was proved by the EIA that 4.2 mg/l of human NGF was produced.

The culture solution thus obtained was centrifuged, and APMSF was added to 2.3 liter of the resulting culture supernatant to a final concentration of 0.1 mM. The solution thus obtained was adsorbed into an S-Sepharose column (2.6 cm X 14 cm) equilibrated with 0.1 M phosphate buffer(pH 6.0)-1 mM EDTA-0.1% CHAPS, and washed with the same buffer, followed by elution with 50 mM Tris-HCl(pH 7.5)-0.7 M NaCl-1 mM EDTA-0.1% CHAPS. The fractions containing human NGF were collected and concentrated about 30-fold with a Diaflow cell (Type YM10, Amicon). The resulting concentrated solution was purified by gel permeation chromatography with a Sephacryl S-100HR column (200 ml, 1.6 cm X 100 cm) equilibrated with 20 mM Tris-HCl(pH 7.4)-0.5 M NaCl-1 mM EDTA-10% glycerol. The fractions containing the human NGF were collected and concentrated about 20-fold on a Centriprep 10 (Amicon). The resulting concentrated solution was subjected to a reverse-phase HPLC to purify the human NGF. Namely, the concentrated solution was applied on Asahipak ODP-50 column (10.0 mm ID X 150 mm L), which was eluted with a linear gradient of 0 to 90% acetonitrile containing 0.1% trifluoroacetic acid to obtain 5.7 mg of a purified human NGF sample. The resulting sample had a purity of 90% or more when measured by reverse-phase HPLC.

Example 7

Cloning of Cell High in Production of Human NGF

The single colony isolation of human NGF producing cell strain CHO-D31-10 obtained in Example 4 was carried out in the following manner.

The cell strain CHO-D31-10 was cultivated in Dulbecco's modified MEM (containing 5% fetal calf serum and 35 µg/ml of proline) containing 10 µM methotrexate, and then the cells were floated with trypsin-EDTA-PBS, followed by dilution with the same medium to a density of 3 cells/ml. A 96-well microtiter plate was seeded with the resulting cells in an amount of 100 µl/well, and the cells were cultivated in the same medium for 2 weeks. A Limbro 24-well culture dish was seeded with 30 clones thus obtained at a density of 5 X $10^4$/well, and the clones were cultivated in the same medium for 5 days. The human NGF in the culture supernatant was assayed by the enzyme immunoassay (EIA) [Behringer, Biochem. Biophys. Res. Commun. 155, 482 (1988)]. Of the resulting clones, CHO-D31-10-2 (IFO 50236, FERM BP-2851) was produced 10 to 15 mg/L of the human NGF in the medium.

Example 8

Isolation of Human NGF (3)

Clone CHO-D31-10-2 obtained in Example 7 was cultivated in Dulbecco's modified minimum essential medium (DMEM) containing 5% fetal calf serum, 35 µg/ml of proline, 50 IU/ml of streptomycin and 10 µM mesothorexate under an atmosphere of 5% carbon dioxide at 37°C for 7 days. Consequently, it was proved by the EIA that 13.7 mg/l of human NGF was produced in the medium.

The culture solution was centrifuged, and APMSF was added to 2.4 liter of the resulting culture supernatant to a final concentration of 0.1 mM. The solution thus obtained was adjusted to pH 6.0 with 0.2 N acetic acid, followed by centrifugation. The supernatant thus obtained was adsorbed onto an S-Sepharose column (2.6 cm X 14 cm) equilibrated with 0.1 M phosphate buffer(pH 6.0)-1 mM EDTA, and washed with 0.1 M phosphate buffer(pH 6.0)-0.15 M NaCl-1 mM EDTA-10% glycerol, followed by elution with 50 mM Tris-HCl(pH 7.5)-0.7 M NaCl-1 mM EDTA-10% glycerol. The fractions containing the human NGF were collected and concentrated about 30-fold with a Diaflow cell (Type YM10, Amicon). The resulting concentrated solution was purified by gel permeation chromatography with a Sephacryl S-100HR column (200 ml, 1.6 cm X 100 cm) equilibrated with 20 mM Tris-HCl(pH 7.4)-0.5 M NaCl-1 mM EDTA-10% glycerol. The fractions containing the human NGF were collected and concentrated about 10-fold on a Centriprep 10 (Amicon). The resulting concentrated solution was subjected to a reverse-phase HPLC to purify the human NGF. Namely, the concentrated solution was applied on Asahipak ODP-50 column (10.0 mm ID X 250 mm L) column, which was eluted with a linear gradient of 0 to 90% acetonitrile containing 0.1% trifluoroacetic acid to obtain 5 mg (calculated from amino acid analysis) of a purified human NGF sample. The purity of the purified human NGF thus obtained was calculated to be about 90% by reverse-phase HPLC.

Table 5 shows the amino acid composition of the purified human NGF thus obtained.

The C-terminal amino acid of the purified human NGF which was examined by hydrazinolysis (previously described) was alanine. Accordingly, it became clear that the above human NGF consisted of 120 amino acids. Valine was also detected as the minor C-terminal amino acid.

In accordance with the method described in Journal of Neuroscience Research 17, 25 (1987), the activity of the purified human NGF was assayed using cell PC12. As a result, the purified human NGF exhibited an activity similar to that of mouse 2.5S NGF (Wako Pure Chemical Industries).

Table 5

| Amino Acid Composition | | |
|---|---|---|
| Amino acid | Experimental 1) value | Theoretical 2) value |
| Asp + Asn | 12.7 | 13 |
| Thr | 9.3 | 10 |
| Ser | 8.5 | 11 |
| Glu + Gln | 6.4 | 6 |
| Pro | 2.5 | 3 |
| Gly | 7.3 | 7 |
| Ala | 6.5 | 7 |
| Half Cys 3) | 5.9 | 6 |
| Val | 12.1 | 13 |
| Met | 2.0 | 2 |
| Ile | 6.0 | 6 |
| Leu | 3.3 | 3 |
| Tyr | 2.1 | 2 |
| Phe | 7.2 | 7 |
| Lys | 9.0 | 9 |
| His | 3.9 | 4 |
| Arg | 7.1 | 8 |
| Trp | 2.8 | 3 |
| Total | | 120 |

1) Calculated taking Lys as 13.
2) Calculated from the amino acid sequence deduced from the nucleotide sequence of the human NGF gene.
3) Calculated as cysteic acid produced by performic acid oxidation and hydrolysis.

## Example 9

### Isolation of Human NGF (4)

Cell strain CHO-D31-10 obtained in Example 4 was cultivated in a manner similar to that of Example 3. The resulting culture supernatant was centrifuged to remove impurities, and then cooled with ice, followed by addition of ammonium sulfate. A precipitate produced when ammonium sulfate was added so as to give 50% saturation was separated by centrifugation. This precipitate was dissolved in a 1/100 volume of 20 mM Tris-HCl (pH 7.4). The amount of human NGF contained therein was assayed by the EIA. As a result, 90% or more NGF was recovered in this fraction. This fraction was passed through a $Cu^{2+}$ column (a Chelating Sepharose 4B column to which $Cu^{2+}$ was chelated, Pharmacia) and washed with 0.5 M NaCl-0.1 M sodium phosphate (pH 6.5). Then, a linear gradient of imidazole from 0 to 30 mM was applied thereto to elute the human NGF. This fraction was concentrated and subjected to the reverse-phase HPLC shown in Example 6, whereby the human NGF could be isolated.

Example 10

The precipitate obtained in Example 9 by ammonium sulfate precipitation was passed through a hydrophobic chromatocolumn (Phenyl Sepharose CL-4B, Pharmacia). The column was washed with distilled water, and a linear gradient of 0 to 50% acetonitrile was applied thereto, thereby eluting human NGF. The eluate was subjected to the reverse-HPLC described in Example 6, whereby high pure human NGF could be obtained similarly.

Example 11

Analysis of Disulfide Bonds in Molecule

Using the purified human NGF obtained in Example 5, the positions of disulfide bonds in its molecule were determined.

To 530 µg of the purified human NGF lyophilized were added 0.2 ml of 0.9% NaCl and 0.8 ml of 0.01 M HCl to dissolve it. The pH of the resulting solution was 2.2. 10.6 µl of a 1 mg/ml pepsin (Sigma) solution was added thereto so as to give a weight ratio of 1/50, followed by reaction at 37°C for 22 hours. Then, 950 µl of the reaction solution was collected, and 50 µl of 250 mM phosphate buffer (pH 6.0) was added thereto to terminate the reaction, thereby obtaining a peptic digest.

The peptic digest was subjected to HPLC, and peptide mapping was carried out. Using a TSK-Gel ODS-120T column (0.45 X 25 cm, Toso), the mixture of 0.05% TFA (A) and 99.95% acetonitrile-0.05% TFC (B) was made to flow therethrough according to the following elution program. The flow rate was 1 ml/min, and the detecting wavelengths were 220 nm and 280 nm.

| Time (min) | % A | % B |
|---|---|---|
| 0 | 98 | 2 |
| 1 | 87 | 13 |
| 35 | 73 | 27 |
| 40 | 40 | 60 |

On the other hand, 10 µl of a 100 mM DTT solution was added to 50 µl of the peptic digest, followed by leaving at room temperature for 3 hours to reduce disulfide bonds. The sample whose disulfide bonds were reduced was similarly subjected to HPLC, and peptide mapping was carried out.

The peptide having disulfide bonds was fixed by comparison of these two peptide mappings, and then this peptide was isolated to analyze a gas-phase protein sequencer-terminal (ABI) amino acid sequence.

The results revealed that this peptide included the following sequence-1, sequence-2 and sequence-3, and that the peptide was a large peptide having three disulfide bonds.

Sequence-1

```
13    15                20
S - V - C - D - S - V - S - V
```

Sequence-2

```
54                58      60                      65
F - E - T - K - C - R - D - P - N - P - V -D - S - G -

        70          73
C - R - G - I - D - S -
```

Sequence-3

```
 102           105                      110                      115
  I - R - I - D - T - A - C - V - C - V - L - S - R - K - A
               120
- V - R - R - A
```

This peptide (2060 pmoles) was concentrated to dryness, and then dissolved in 0.3 ml of 50 mM sodium acetate (pH 6.0). To this solution was added 0.48 μg (14 pmoles) of thermolysin (Wako Pure Chemical Industries), followed by reaction at 37°C for 20 hours. Then, the resulting reaction solution was subjected to HPLC and peptide mapping was carried out, under the same conditions as described above with the exception that the following elution program was used.

| Time (min) | % A | % B |
|---|---|---|
| 0 | 100 | 0 |
| 1 | 97 | 3 |
| 25 | 82 | 18 |
| 30 | 60 | 40 |
| 31 | 100 | 0 |

Further, in order to determine the positions of the disulfide bonds, a sample obtained by reducing the above peptide with DTT was subjected to HPLC similarly, and peptide mapping was carried out. By comparison of the peptide mappings, three fragments (fragment-1, fragment-2 and fragment-3) were fixed to obtain each.

The amino-terminal amino acid sequences of the fragment-1, fragment-2 and fragment-3 were analyzed. The results are shown in Table 6.

On the other hand, these fragments were oxidized with performic acid to convert cysteine residues into cysteic acid residues, followed by amino acid analysis. As a result, two cysteic acid residues were detected in each fragment.

Table 6

| Cycle | Fragment-1<br>Detected amino acid (pmole) | Fragment-2<br>Detected amino acid (pmole) | Fragment-3<br>Detected amino acid (pmole) |
|---|---|---|---|
| 1 | Ser(60) | Phe(90) Ala(86) | Val(115) |
| 2 | Val(43) Tyr(43) | Glu(78) | Asp(70) |
| 3 | - - | Thr(45) | Ser(8) |
| 4 | Asp(43) Thr(28) | Lys(62) | Gly(47) |
| 5 | Ser(17) | - | - |
| 6 | | Arg(78) | Arg(27) |
| 7 | | Asp(49) | Gly(24) |
| 8 | | Pro(33) | - |
| 9 | | Asn(31) | - |
| 10 | | Pro(11) | - |
| Structure | $S^{13}$-V-$C^{15}$-D-$S^{17}$<br>\|<br>$S^{78}$-Y-$C^{80}$-$T^{81}$ | $F^{54}$-E-T-K-$C^{58}$-R-D-P-N-$P^{63}$<br>\|<br>$A^{107}$-$C^{108}$ | $V^{64}$-D-S-G-$C^{68}$-R-$G^{70}$<br>\|<br>$V^{109}$-$C^{110}$ |

From these results, it was concluded that the disulfide bonds of the purified human NGF were positioned at $Cys^{15}$-$Cys^{80}$,$Cys^{58}$-$Cys^{108}$ and $Cys^{68}$-$Cys^{110}$.

This disulfide bond form is shown in Fig. 7.

## Claims

**Claims for the following Contracting States : AT, BE, CH/LI, DE, DK, FR, GB, GR, IT, LU, NL, SE**

1. A Chinese hamster ovary (CHO) cell which is transformed with a vector containing a gene coding for a human nerve growth factor protein and is cloned.

2. The transformed CHO cell in accordance with claim 1, which is CHO-D31-10 (FERM BP-2674).

3. The transformed CHO cell in accordance with claim 1, which is CHO-D31-10-2 (FERM BP-2851).

4. The CHO cell according to claim 1, wherein said protein comprises 120 amino acids.

5. The CHO cell according to claim 1, wherein said protein has an amino acid sequence and disulfide bonds as shown in Fig. 7.

6. A method for producing a human nerve growth factor protein which comprises cultivating and CHO cell according to any of claims 1-5.

**Claims for the following Contracting State : ES**

1. A method for producing a Chinese hamster ovary (CHO) cell which is transformed with a vector containing a gene coding for a human nerve growth factor protein and is cloned which method comprises transforming a CHO host cell with said vector.

2. A method in accordance with claim 1, wherein the transformed animal cell is CHO-D31-10 (FERM BP-2674).

3. A method in accordance with claim 1, wherein the transformed animal cell is CHO-D31-10-2 (FERM BP-2851).

4. A method in accordance with claim 1, wherein said protein comprises 120 amino acids.

5. A method in accordance with claim 1, wherein said protein has an amino acid sequence and disulfide bonds as shown in Fig. 7.

6. A method for producing a human nerve growth factor protein which comprises cultivating and CHO cell produced according to a method of any of claims 1-5.

**Patentansprüche**
**Patentansprüche für folgende Vertragsstaaten : AT, BE, CH/LI, DE, DK, FR, GB, GR, IT, LU, NL, SE**

1. Eierstockzelle des chinesischen Hamsters (CHO), die mit einem Vektor transformiert ist, der ein Gen enthält, das für ein menschliches Nervenwachstumsfaktor-Protein kodiert und kloniert ist.

2. Transformierte CHO-Zelle gemäß Anspruch 1, die CHO-D31-10 (FERM BP-2674) ist.

3. Transformierte CHO-Zelle gemäß Anspruch 1, die CHO-D31-10-2 (FERM BP-2851) ist.

4. CHO-Zelle gemäß Anspruch 1, in der das Protein 120 Aminosäuren umfaßt.

5. CHO-Zelle gemäß Anspruch 1, in der das Protein eine wie in Fig. 7 dargestellte Aminosäuresequenz und Disulfidbindungen besitzt.

6. Verfahren zum Herstellen eines menschlichen Nervenwachstumsfaktor-Proteins, welches das Kultivieren einer CHO-Zelle gemäß einem der Ansprüche 1-5 umfaßt.

**Patentansprüche für folgenden Vertragsstaat : ES**

1. Verfahren zum Herstellen einer Eierstockzelle des chinesischen Hamsters (CHO), die mit einem Vektor transformiert ist, der ein Gen enthält, das für ein menschliches Nervenwachstumsfaktor-Protein kodiert und kloniert ist, wobei das Verfahren das Transformieren einer CHO-Wirtszelle mit dem Vektor umfaßt.

2. Verfahren gemäß Anspruch 1, bei welchem die transformierte Tierzelle CHO-D31-10 (FERM BP-2674) ist.

3. Verfahren gemäß Anspruch 1, bei welchem die transformierte Tierzelle CHO-D31-10-2 (FERM BP-2851) ist.

4. Verfahren gemäß Anspruch 1, bei welchem das Protein 120 Aminosäuren umfaßt.

5. Verfahren gemäß Anspruch 1, bei welchem das Protein eine wie in Fig. 7 dargestellte Aminosäuresequenz und Disulfidbindungen besitzt.

6. Verfahren zum Herstellen eines menschlichen Nervenwachstumsfaktor-Proteins, welches das Kultivieren einer gemäß einem der Ansprüche 1-5 hergestellten CHO-Zelle umfaßt.

## Revendications

**Revendications pour les Etats contractants suivants : AT, BE, CH/LI, DE, DK, FR, GB, GR, IT, LU, NL, SE**

1. Cellule d'ovaire de hamster chinois (CHO) qui est transformée avec un vecteur contenant un gène codant une protéine qui est un facteur humain de croissance des nerfs, et qui est clonée.

2. Cellule CHO transformée conforme à la revendication 1, qui est CHO-D31-10 (FERM BP-2674).

3. Cellule CHO transformée conforme à la revendication 1, qui est CHO-D31-10-2 (FERM BP-2851).

4. Cellule CHO conforme à la revendication 1, dans laquelle ladite protéine comporte 120 résidus d'acides aminés.

5. Cellule CHO conforme à la revendication 1, dans laquelle ladite protéine présente la séquence d'acides aminés et les ponts disulfures représentés sur la figure 7.

6. Procédé de production d'une protéine qui est un facteur humain de croissance des nerfs, lequel procédé comporte le fait de cultiver des cellules CHO conformes à l'une des revendications 1 à 5.

**Revendications pour l'Etat contractant suivant : ES**

1. Procédé d'obtention d'une cellule d'ovaire de hamster chinois (CHO), transformée avec un vecteur contenant un gène codant une protéine qui est un facteur humain de croissance des nerfs et clonée, lequel procédé comporte le fait de transformer une cellule CHO hôte avec ledit vecteur.

2. Procédé conforme à la revendication 1, dans lequel la cellule animale transformée est CHO-D31-10 (FERM BP-2674).

3. Procédé conforme à la revendication 1, dans lequel la cellule animale transformée est CHO-D31-10-2 (FERM BP-2851).

4. Procédé conforme à la revendication 1, dans lequel ladite protéine comporte 120 résidus d'acides aminés.

5. Procédé conforme à la revendication 1, dans lequel ladite protéine présente la séquence d'acides aminés et les ponts disulfures représentés sur la figure 7.

6. Procédé de production d'une protéine qui est un facteur humain de croissance des nerfs, lequel procédé comporte le fait de cultiver des cellules CHO obtenues selon un procédé conforme à l'une des revendications 1 à 5.

Fig. 1-1

```
SmaI
CCCGGGTTACGCCTGTTGTCCCGGTATAACCATTGCTAGCACACCCTTTCCCTCTCAGAAGTGCCCCG

GTTTGAATGAAACCTCTTCGTGATCCCCTTGGGAGGTCAACTCTGAGGGACCCAGAAACTGCCTTTTGA

                                                                   →signal
                                                                   MetSerMet
CTGCATTTAGTACTCCATGAAGTCACCCTCATTTCTTTTTCATTCCAGGTGCATAGCGTAATGTCCATG

            BclI                                     →Pro
LeuPheTyrThrLeuIleThrAlaPheLeuIleGlyIleGlnAlaGluProHisSerGluSerAsnVal
TTGTTCTACACTCTGATCACAGCTTTTCTGATCGGCATACAGGCGGAACCACACTCAGAGAGCAATGTC

ProAlaGlyHisThrIleProGlnValHisTrpThrLysLeuGlnHisSerLeuAspThrAlaLeuArg
CCTGCAGGACACACCATCCCCCAAGTCCACTGGACTAAACTTCAGCATTCCCTTGACACTGCCCTTCGC

ArgAlaArgSerAlaProAlaAlaAlaIleAlaAlaArgValAlaGlyGlnThrArgAsnIleThrVal
AGAGCCCGCAGCGCCCCGGCAGCGGCGATAGCTGCACGCGTGGCGGGGCAGACCCGCAACATTACTGTG

AspProArgLeuPheLysLysArgArgLeuArgSerProArgValLeuPheSerThrGlnProProArg
GACCCCAGGCTGTTTAAAAAGCGGCGACTCCGTTCACCCCGTGTGCTGTTTAGCACCCAGCCTCCCCGT

GluAlaAlaAspThrGlnAspLeuAspPheGluValGlyGlyAlaAlaProPheAsnArgThrHisArg
GAAGCTGCAGACACTCAGGATCTGGACTTCGAGGTCGGTGGTGCTGCCCCCTTCAACAGGACTCACAGG

         →mature
SerLysArgSerSerSerHisProIlePheHisArgGlyGluPheSerValCysAspSerValSerVal
AGCAAGCGGTCATCATCCCATCCCATCTTCCACAGGGGCGAATTCTCGGTGTGTGACAGTGTCAGCGTG

TrpValGlyAspLysThrThrAlaThrAspIleLysGlyLysGluValMetValLeuGlyGluValAsn
TGGGTTGGGGATAAGACCACCGCCACAGACATCAAGGGCAAGGAGGTGATGGTGTTGGGAGAGGTGAAC

IleAsnAsnSerValPheLysGlnTyrPhePheGluThrLysCysArgAspProAsnProValAspSer
ATTAACAACAGTGTATTCAAACAGTACTTTTTTGAGACCAAGTGCCGGGACCCAAATCCCGTTGACAGC
```

Fig. 1−2

```
GlyCysArgGlyIleAspSerLysHisTrpAsnSerTyrCysThrThrThrHisThrPheValLysAla
GGGTGCCGGGGCATTGACTCAAAGCACTGGAACTCATATTGTACCACGACTCACACCTTTGTCAAGGCG

LeuThrMetAspGlyLysGlnAlaAlaTrpArgPheIleArgIleAspThrAlaCysValCysValLeu
CTGACCATGGATGGCAAGCAGGCTGCCTGGCGGTTTATCCGGATAGATACGGCCTGTGTGTGTGTGCTC

SerArgLysAlaValArgArgAla
AGCAGGAAGGCTGTGAGAAGAGCCTGACCTGCCGACACGCTCCCTCCCCCTGCCCCTTCTACACTCTCC

  ApaI
TGGGCCC
```

Fig. 2

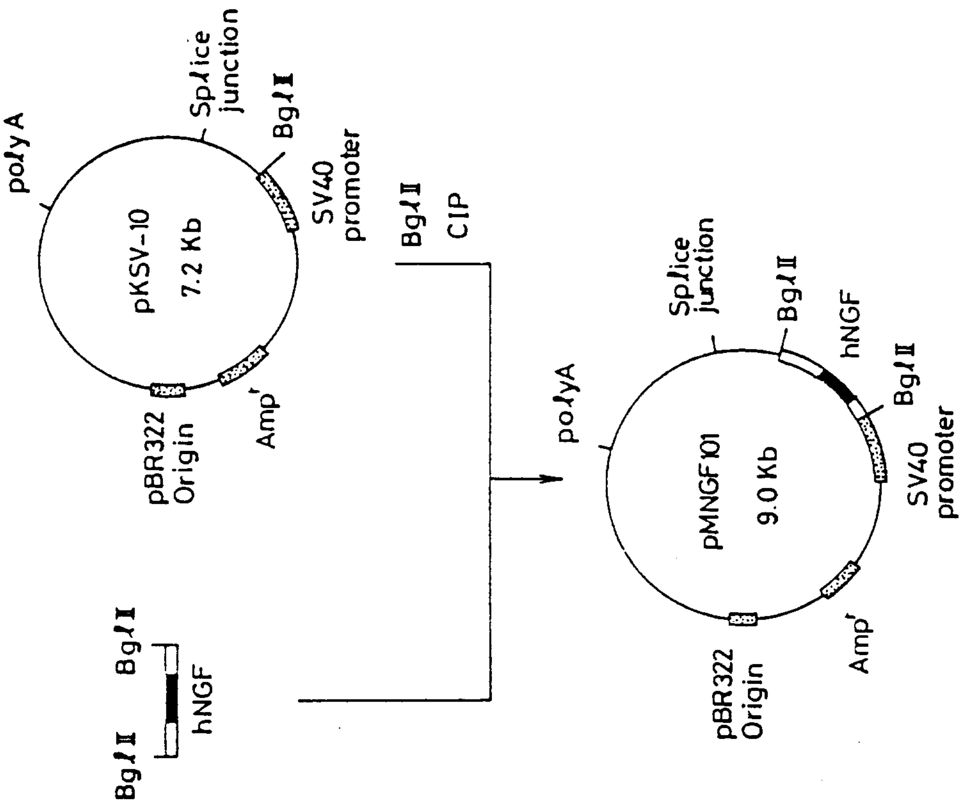

polyA
Splice junction
BglII
SV40 promoter
pKSV-10
7.2 Kb
pBR322 Origin
Ampr
BglII
CIP
BglII
hNGF
BglII
polyA
Splice junction
BglII
hNGF
BglII
pMNGF101
9.0 Kb
SV40 promoter
pBR322 Origin
Ampr

Fig. 3

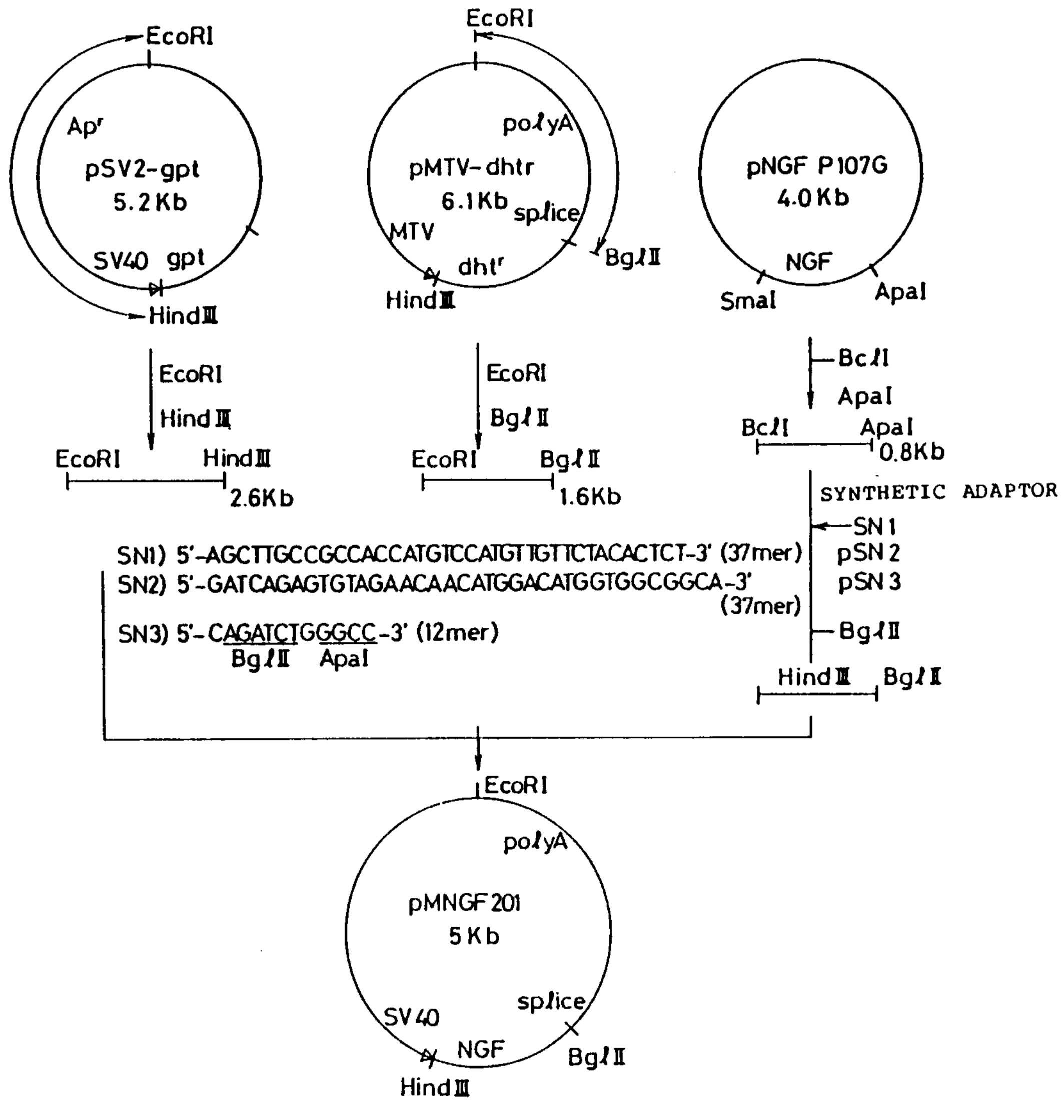
EcoRI
Ap^r
pSV2-gpt
5.2 Kb
SV40 gpt
HindIII
EcoRI
polyA
pMTV-dhtr
6.1 Kb
splice
MTV
dht^r
BglII
HindIII
pNGF P107G
4.0 Kb
NGF
SmaI
ApaI
EcoRI
Hind III
EcoRI
HindIII
2.6Kb
EcoRI
BglII
EcoRI
BglII
1.6Kb
BclI
ApaI
BclI
ApaI
0.8Kb
SYNTHETIC ADAPTOR
SN1
pSN2
pSN3
BglII
SN1) 5'-AGCTTGCCGCCACCATGTCCATGTTGTTCTACACTCT-3' (37mer)
SN2) 5'-GATCAGAGTGTAGAACAACATGGACATGGTGGCGGCA-3' (37mer)
SN3) 5'-CAGATCTGGGCC-3' (12mer)
BglII ApaI
HindIII BglII
EcoRI
polyA
pMNGF201
5 Kb
splice
SV40
NGF
BglII
HindIII

Fig. 4

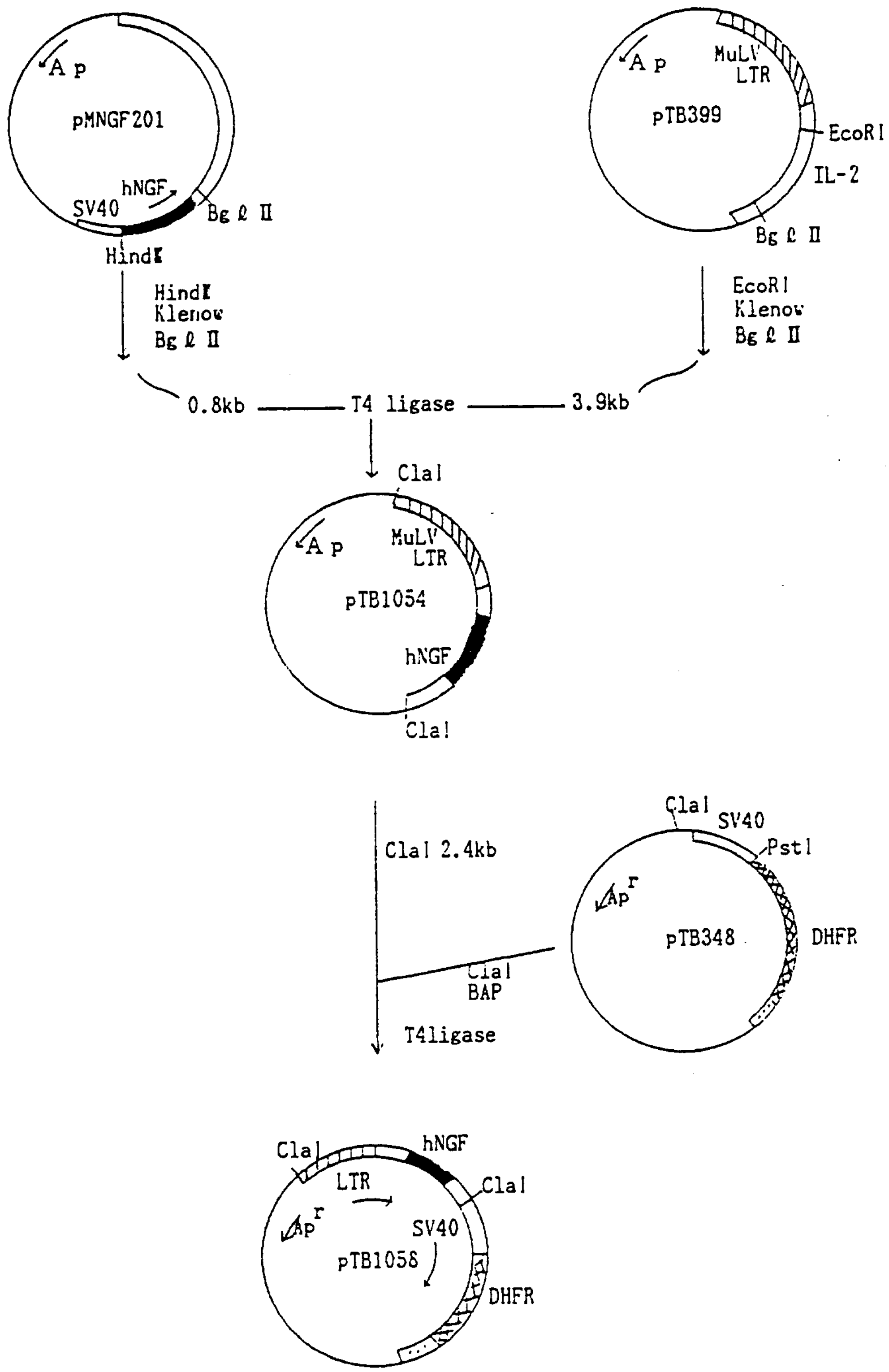
Ap
pMNGF201
hNGF
SV40
Bgℓ II
HindⅢ
HindⅢ
Klenow
Bgℓ II
Ap
MuLV
LTR
pTB399
EcoRI
IL-2
Bgℓ II
EcoRI
Klenow
Bgℓ II
0.8kb
T4 ligase
3.9kb
ClaI
Ap
MuLV
LTR
pTB1054
hNGF
ClaI
ClaI 2.4kb
ClaI
BAP
T4ligase
ClaI
SV40
PstI
Apr
pTB348
DHFR
ClaI
hNGF
LTR
ClaI
Apr
SV40
pTB1058
DHFR

Fig. 5

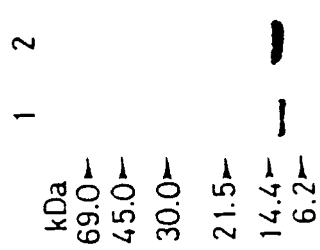

Fig. 6

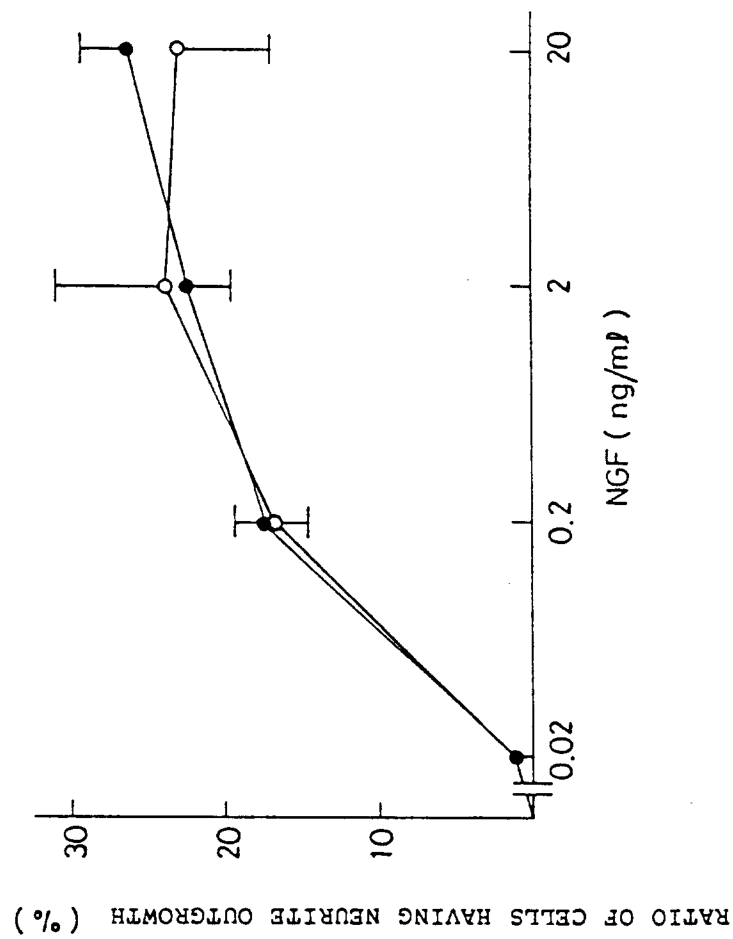
RATIO OF CELLS HAVING NEURITE OUTGROWTH (%)
30
20
10
0.02
0.2
2
20
NGF ( ng/ml )

Fig. 7

```
                                           10                                           20
Ser-Ser-Ser-His-Pro-Ile-Phe-His-Arg-Gly-Glu-Phe-Ser-Val-Cys-Asp-Ser-Val-Ser-Val-
                                           30                                           40
Trp-Val-Gly-Asp-Lys-Thr-Thr-Ala-Thr-Asp-Ile-Lys-Gly-Lys-Glu-Val-Met-Val-Leu-Gly-
                                           50                                           60
Glu-Val-Asn-Ile-Asn-Asn-Ser-Val-Phe-Lys-Gln-Tyr-Phe-Phe-Glu-Thr-Lys-Cys-Arg-Asp-
                                           70                                           80
Pro-Asn-Pro-Val-Asp-Ser-Gly-Cys-Arg-Gly-Ile-Asp-Ser-Lys-His-Trp-Asn-Ser-Tyr-Cys-
                                           90                                           100
Thr-Thr-Thr-His-Thr-Phe-Val-Lys-Ala-Leu-Thr-Met-Asp-Gly-Lys-Gln-Ala-Ala-Trp-Arg-
                                           110
Phe-Ile-Arg-Ile-Asp-Thr-Ala-Cys-Val-Cys-Val-Leu-Ser-Arg-Lys-Ala-Val-Arg-Arg-Ala
```